# EUROPÄISCHE PATENTSCHRIFT

(11) **EP 0 914 061 B1**
(45) Veröffentlichungstag und Bekanntmachung des Hinweises auf die Patenterteilung: **12.03.2003**
(21) Anmeldenummer: 98919142.4
(22) Anmeldetag: 24.03.1998
(51) Int. Cl.: A61B 8/06, A61B 8/12

(54) **VORRICHTUNG FÜR DIE BEOBACHTUNG VON GEFÄSSEN, INSBESONDERE BLUTGEFÄSSEN**
DEVICE FOR OBSERVING VESSELS, SPECIALLY BLOOD VESSELS
DISPOSITIF POUR OBSERVER DES VAISSEAUX, NOTAMMENT DES VAISSEAUX SANGUINS

(30) Priorität: 25.03.1997 DE 19712572; 23.01.1998 DE 19802474
(43) Veröffentlichungstag der Anmeldung: 12.05.1999
(73) Patentinhaber: DWL Elektronische Systeme GmbH, D-78354 Sipplingen (DE)
(72) Erfinder: GRUND, Karl, Ernst, D-72070 Tübingen (DE); KIMMEL, Martin, D-73732 Esslingen (DE); DENNER, Dieter, D-78354 Sipplingen (DE); WIDENHORN, Gerold, D-88662 Überlingen (DE); ZOLONDZ, Jürgen, D-78315 Radolfzell (DE)
(74) Vertreter: Behrmann, Niels, Dipl.-Ing.
(86) Internationale Anmeldenummer: EP9801733
(87) Internationale Veröffentlichungsnummer: WO98042259

(56) Entgegenhaltungen:
- EP-A- 0 668 052
- DE-A- 3 838 396
- DE-A- 3 934 644
- US-A- 4 582 067
- US-A- 5 394 878

## Beschreibung

Die vorliegende Erfindung betrifft eine Vorrichtung für die Beobachtung von Gefäßen, insbesondere Blutgefäßen, am Körper, in Körperhöhlen und Hohlorganen.

Aus dem Stand der Technik ist es bekannt, zur Untersuchung von Körperhöhlen oder Hohlorganen im Körperinneren, etwa dem Magen oder dem Darmtrakt, ein Endoskop einzuführen, um durch dessen Bilddarstellung einen optischen Eindruck von einem zu diagnostizierenden Zustand im jeweiligen Körperbereich zu erhalten. Technik, Aufbau und Funktionsweise derartiger Endoskope sind bekannt, wobei insbesondere die moderneren einen mittels Lichtleitfasern realisierten Endoskop-Leiter zur Einführung in den Körper aufweisen und das so erreichte Bild über einen elektronischen Bildsensor erfaßt und zur Anzeige auf einem (Video-) Bildschirm elektronisch aufbereitet wird. Auch gibt es Endoskope, die am Eingriffsende den Bildsensor (z.B. CCD) aufweisen.

Bei der praktischen Durchführung der Endoskopie stellt sich jedoch häufig das Problem, daß an für eine Diagnose oder Behandlung konkret relevanten Stellen, z.B. einem Geschwür, (Blut-) Gefäße vorhanden sind, welche besonderer Aufmerksamkeit bzw. extremer Vorsicht bei Eingriffen oder einer zielgerichteten Behandlung bedürfen, um eine Blutungsgefahr zu verringern. Da zudem derartige Blutgefäße von Gewebe oder Organwänden der jeweils betrachtenden Körperhöhle umgeben sind, sind diese in der Regel durch die rein endoskopische Betrachtung visuell nicht erkennbar.

Zwar ist es ebenfalls nach dem Stand der Technik bekannt, generell zur Untersuchung von Blutflüssen und somit auch zur Detektion von Blutgefäßen Techniken der Ultraschall-Sonographie anzuwenden, dies bietet jedoch insbesondere bei einer Arbeit im Körperinneren ganz erhebliche Schwierigkeiten und ist daher in der klinischen Praxis bislang nicht nutzbar.

Eine Vorrichtung nach dem Oberbegriff des Hauptanspruchs ist aus der US 4,582,067 bekannt; eine weitere Vorrichtung zur Beobachtung von Gefäßen, insbesondere von Blutgefäßen, Körperöffnungen od. dgl., ist aus der EP 0 668 052 A2 bekannt, basierend auf Ultraschall diagnostischen Verfahren.

Problematisch ist es jedoch nach wie vor, dass diese bekannten Vorrichtungen dem Bediener Darstellungen der mittels der Ultraschall-Einheiten gewonnenen Informationen und Erkenntnisse nicht in übersichtlicher oder für einen operativen Ansatz brauchbarer Weise darbieten.

Aufgabe der vorliegenden Erfindung ist es daher, die gattungsbildende Vorrichtung im Hinblick auf die Flexibilität und Einfachheit der visuellen Darstellung der Ausgangssignale der Ultraschall-Einheit zu verbessern.

Die Aufgabe wird durch die Vorrichtung nach dem Hauptanspruch gelöst; vorteilhafte Weiterbildungen der Erfindung sind in den Unteransprüchen beschrieben.

Vorteilhaft gestattet dabei das Vorsehen einer UltraschallSonde für eine (bevorzugt mehrkanalige) Doppier-Ultraschall-Auswertung am eingriffsseitigen Ende des bevorzugt als Endoskopleiter ausgeführten Trägers die gleichzeitige Überwachung auf (Blut-)gefäße bzw. das Erzeugen entsprechender, strömungsabhängiger Bildinformation bei der Betrachtung des erfaßten Bildes, und die erfindungsgemäß ausgebildete Video-Ausgabeeinheit ermöglicht die simultane, visuelle Überwachung dieser Vorgänge mit einem einzigen Blick auf einem gemeinsamen Ausgabebildschirm.

In für die klinische Praxis bedeutsamer Weise wird somit die Beobachtung bzw. Behandlung zweier diagnostisch zusammenhängender Tatbestände ermöglicht und unterstützt, ohne daß mehrere Geräte simultan bedient und beobachtet werden müssen, und ohne daß eine Ablenkung durch zusätzlichen Bedienaufwand erfolgt.

Zwar ist es im Rahmen der Erfindung bevorzugt, diese im Zusammenhang mit einer Endoskopieeinrichtung zu benutzen, bei welcher diese als Trägereinrichtung auch für den Ultraschallwandler verwendet wird; die Erfindung ist jedoch nicht auf diese Ausführungsform beschränkt. So liegt es generell im Rahmen der Erfindung, eine nahezu beliebige, elektrische Bildaufnahmevorrichtung an einem entsprechenden Träger zusammen mit dem Ultraschallwandler einzusetzen. Beispielsweise ist die Erfindung geeignet auch im Zusammenhang mit einem Operationsmikroskop zu verwenden, bei welchem das Bilderfassungselement zum Übertragen des Mikroskopbildes der Mikroskopoptik zugeordnet ist, während eingriffsseitig am Mikroskop der Ultraschallwandler sitzen kann; insoweit wirkt das Mikroskop als erfindungsgemäße Trägereinrichtung.

Weitere, darüber hinausgehende Einsatzbereiche wären dann etwa die Laparoskopie (Bauchspiegelung) bzw. die Neurochirurgie. Generell erstreckt sich damit die Erfindung auf entsprechend zugehörige Video- bzw. Grafikquellen.

Vorteilhafte Weiterbildungen der Erfindung sind in den Unteransprüchen beschrieben.

So wird insbesondere durch die mehrkanalige, tiefenweise gestaffelte Auflösung der Ultraschall-Dopplerüberwachung eine praktisch äußerst nützliche Tiefenfeststellung eines jeweiligen Blutgefäßes ermöglicht. Gerade in der Durchführung möglicher operativer Eingriffe hat es sich nämlich als bedeutsam herausgestellt, vorab eine Gewissheit dafür zu haben, ob überhaupt ein Blutfluß vorhanden ist, und dann, in welcher Tiefe ein solches Blutgefäß liegt.

Gerade durch die erfindungsgemäße Kombination der Dopplersonde mit einer Endoskopspitze, welche die Betrachtungsebene für das visuelle Endoskopie-Bild festlegt, liegt die Ultraschallsonde in einer fest definierten Position und gestattet somit eine zuverlässige Abbildung der möglichen (Blut-) Flußverhältnisse im Organgewebe oder der Wand relativ zum Endoskopkopf.

Vorteilhaft gestattet die Erfindung zudem das bedarfsgerechte Umschalten zwischen einer reinen Endoskopie-Darstellung und einer in der erfindungsgemäßen Weise spezifizierten Mischdarstellung, insbesondere für den Fall, daß an einer als besonders relevant angesehenen Eingriffsposition ein Gefäß vermutet wird.

Weiterbildungsgemäß basiert zudem die Video-Ausgabeeinheit auf einem digitalen Videomischer. Hierdurch kann dann nicht nur in einfacher Weise das -- durch die Auswertung bzw. Berechnung ohnehin digitale -- Dopplerbild einfach verarbeitet werden; darüber hinaus ist auch eine einfache und bedarfsgerechte Anpassung des Endoskopbildes, etwa mit der weiterbildungsgemäß vorzusehenden Skaliereinheit zur Verkleinerung bzw. Vergrößerung, möglich.

Weiter vorteilhaft ist die erfindungsgemäße Vorrichtung zum Einspeisen bzw. Ausgeben von Bildsignalen einer Mehrzahl verschiedener, gängiger Bildnormen, insbesondere TV-Bildnormen eingerichtet. Dabei sind weiterbildungsgemäß Bildsignale insbesondere auch parallel, d. h. gleichzeitig in verschiedenen Normen, ausgebbar.

Gemäß einer weiteren, bevorzugten Ausführungsform der Erfindung ist es zudem möglich, den Ultraschallwandler selbst, d. h. den Sondenkopf, am Eingriffsende des Trägers (z.B. des Endoskopschlauchs) in seiner Position zu verändern. Auf diese Weise wird vorteilhaft eine Signaloptimierung bzw. Anpassung an jeweils spezifische Beobachtungsumstände erreicht.

Vorteilhaft liegt es zudem im Rahmen der Erfindung, den Sondenkopf rotierend vorzusehen und/oder eine Linse zur Veränderung des Schallfeldes aufzusetzen, oder den Sondenkopf beweglich in Form einer steuerbaren Sonde auszuführen.

Insgesamt entstehen somit durch die Erfindung völlig neue Möglichkeiten der beispielsweise endoskopgestützten (bzw. allgemein: microinvasiven) Erforschung, Diagnose und Therapie. Erstmals wird es nun einem Bediener ermöglicht, auf einen Blick eine endoskopische Untersuchung vorzunehmen, und gleichzeitig an potentiell blutungsgefährdeten Stellen eine Prüfung auf (verdeckte) Blutgefäße vorzunehmen, ohne daß es etwa weiterer (aufwendiger) Techniken oder Bedienschritte bedarf.

Weitere Vorteile, Merkmale und Einzelheiten der Erfindung ergeben sich aus der nachfolgenden Beschreibung von Ausführungsbeispielen sowie anhand der Zeichnungen; diese zeigen in
- Fig. 1:: ein schematisches Blockschaltbild der erfindungsgemäßen Vorrichtung für die Beobachtung von Blutgefäßen in Körperhöhlen und Hohlorganen mit den wesentlichen Funktionskomponenten;
- Fig. 2:: eine Schemaansicht einer beispielhaften Bildschirmdarstellung auf der Video-Ausgabeeinheit gemäß einer bevorzugten Ausführungsform der Erfindung (best mode);
- Fig. 3:: ein Blockschaltbild der Doppler-und Video-Einheit aus Fig. 1;
- Fig. 4:: ein weiter detailliertes Blockschaltbild der Video-Verarbeitungseinheit aus Fig. 3; und
- Fig. 5:: ein Flußdiagramm mit den Verfahrens- bzw. Verarbeitungsschritten zum Erzeugen einer kombinierten Doppler- und Endoskop-Bilddarstellung auf der Video-Ausgabeeinheit gemäß Fig. 2.

Ein handelsübliches, flexibles Endoskop 10 ist in herkömmlicher Weise mit einer Endoskopie-Basiseinheit 12, welche die für die Endoskop-Bilderzeugung notwendige Elektronik aufweist, verbunden. Diese so aufgebaute Endoskopieeinheit 10, 12 entspricht im Hinblick auf ihre Bestandteile und ihre Funktionsweise handelsüblichen Endoskopiesystemen, wobei -- je nach vorgesehenem Anwendungsfall -- Geräte für eine flexible Endoskopie oder aber für eine starre Endoskopie, etwa bei der Laparaskopie, verwendbar sind.

Durch den (in den Fig. nicht näher gezeigten) Instrumentier- bzw. Arbeitskanal des flexiblen Endoskops (Endoskopleiters) 10 ist zusätzlich eine entsprechend ausgeformte, mit einer dünnen, flexiblen Zuleitung versehene Ultraschallsonde 14 an einen Eingriffsort am Endoskopende geführt. Diese Ultraschallsonde ist zum Senden und Empfangen von Ultraschallwellen zur Gefäßerkennung und -beobachtung unter Ausnutzung des Dopplereffektes eingerichtet und über eine entsprechende Zuleitung mit einer Doppler- und Video-Einheit 16 verbunden. Diese Doppler- und Video-Einheit 16 empfängt zusätzlich das Endoskop-Bildsignal (welches z.B. das Ausgangssignal eines Videoendoskops sein kann, welches in geeigneter und ansonsten bekannter Weise von der Basiseinheit 12 zu einem Monitorbild verarbeitet wurde) und erzeugt daraus ein gemeinsames Video-Signal, welches an eine Video-Ausgabeeinheit 18 in der nachfolgend zu beschreibenden Weise ausgegeben wird. Dabei basiert das gemeinsame Video-Signal sowohl auf Bildinformationen, die mit der Ultraschallsonde 14 gewonnen werden, als auch auf dem Endoskopiebild.

Ein schematisch gezeigter, extern angeschlossener Umschalter -- im dargestellten Beispiel ein Fußschalter 20 -- erlaubt es einer Bedienperson, zwischen Modi der Bilddarstellung auf der Ausgabeeinheit 18 umzuschalten. Darüber hinaus ist eine externe Schnittstellen- bzw. Fernbedieneinheit 22 vorgesehen, welche eine zusätzliche, externe Ausgabe und Bearbeitung der durch die erfindungsgemäße Vorrichtung erzeugten Informationen ermöglicht, und über welche ggf. zusätzlich mögliche Steuerbefehle gegeben werden können.

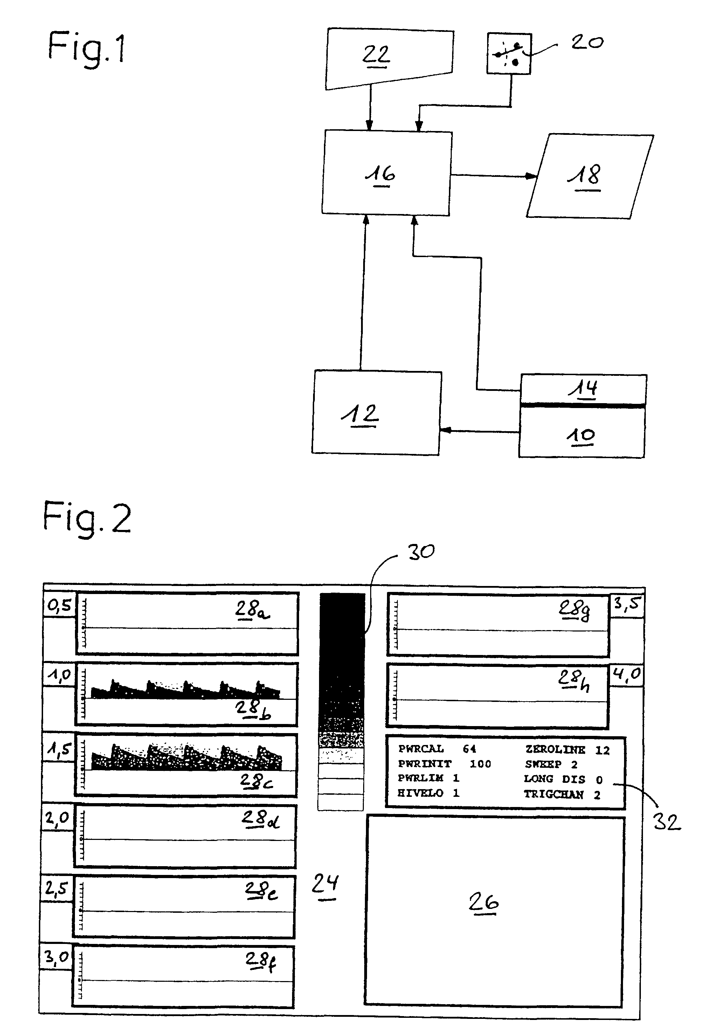

Wie in der Fig. 1 durch das schematisch nebeneinanderliegend angedeutete Funktionsmodul 10, 14 gezeigt, ist die Kombination aus flexiblem Endoskop und ebenso flexibler Ultraschallsonde geeignet, an die jeweils zu beobachtenden Positionen in der Körper- bzw. Organhöhle geführt zu werden, um dort über das Endoskop ein visuelles Bild der jeweiligen Betrachtungsstelle zu erzeugen und mittels der Ultraschallsonde -- bevorzugt mehrkanalig -- eine gleichzeitige Untersuchung der an diese Beobachtungsposition verlaufenden (Blut-) Gefäße zu ermöglichen.

Die Untersuchungs- und Darstellungsmöglichkeiten werden anhand der schemaweisen Video-Bilddarstellung der Fig. 2, die ein typisches Monitor-Ausgabebild zeigt, verdeutlicht.

Innerhalb einer Bildschirmfläche ist im dargestellten Ausführungsbeispiel im rechten unteren Bereich ein Feld bzw. ein Fenster für das eigentliche Endoskopiebild 26 eingerichtet, welches dem durch die Endoskopie-Basiseinheit 12 erzeugten Ausgabesignal entspricht. Darüber hinaus sind auf der Bildschirmfläche 24, da es sich beim dargestellten Ausführungsbeispiel um eine achtkanalige Ultraschall-Dopplereinheit handelt, acht Ultraschalldarstellungen 28ₐ bis 28ₕ abgebildet, die -- in ihrer Eindringtiefe abgestuft --Blutfluß und daher das Vorliegen eines Gefäßes in einer jeweiligen Eindringtiefe zeigen. Genauer gesagt sind bei dem dargestellten Ausführungsbeispiel acht Ultraschall-Eindringtiefen zwischen 0,5 und 4,0 mm eingestellt, erkennbar an den Tiefenanzeigen neben einer jeweiligen Ultraschall-Darstellung, die ein jeweiliges aufbereitetes Ultraschallsignal zeigen. Im Ausführungsbeispiel der Fig. 2 ist bei einer Eindringtiefe von 1,0 sowie 1,5 mm ein Dopplersignal mit einem entsprechend der Herzfrequenz gepulsten Signalverlauf erkennbar, was darauf hindeutet, daß in diesem Tiefenbereich (bezogen auf die Spitze der Ultraschallsonde) ein Blutgefäß existiert.

Die Bilddarstellung des Ultraschall-Dopplersignals in den Fenstern 28 der Fig. 2 ist eine Darstellung eines jeweils ermittelten Frequenzspektrums (Vertikalachse) über der Zeit (Horizontalachse), wobei in ansonsten bekannter Weise für diese Darstellung das Doppler-Empfangssignal einer nachfolgenden Bildverarbeitungs- und Transformationsbehandlung zum Erzeugen des dargestellten Frequenzspektrums über der Zeit unterzogen wurde. Darüber hinaus enthält die Bilddarstellung der Fenster 28 eine Amplitudeninformation, d.h. eine Darstellung einer jeweiligen Signalstärke innerhalb einer Frequenzlinie, durch eine in der Fig. lediglich mittels einer Farb-/Grauskala 30 angedeuteten, amplitudenabhängigen und bevorzugt in dB geeichten farbigen Einfärbung des Dopplersignals. In der dargestellten Abbildung beträgt der Zeitablauf auf der gesamten horizontalen Zeitachse etwa 6 Sekunden.

Auf der Bildschirmfläche 24 ist schließlich ein Textfeld 32 vorgesehen, welches zum Anzeigen aktueller Betriebsparameter -- Ultraschall-Sendeleistung, horizontale Ablenkung, Verstärkung, Wellenform usw. -- geeignet ist.

Mittels des Fußschalters 20 ist zudem die Bilddarstellung auf der Bildschirmfläche 24 in einfacher Weise zwischen der in Fig. 2 gezeigten Anordnung und einer Vollbilddarstellung des Endoskopiebildes 26 umschaltbar, so daß die Bedienperson etwa bei Endoskopie-Vollbilddarstellung eine Untersuchung der betroffenen Körperhöhle vornehmen kann und den Kopf des Endoskops bewegt, bis eine näher zu untersuchende Position erreicht ist. Dann wird die Ultraschallsonde in den interessanten Bereich plaziert, woraufhin dann mittels des Fußschalters 20 auf den in der Fig. 2 gezeigten Darstellungsmodus umgeschaltet wird und für die aktuelle Position des Endoskopleiterkopfes eine zusätzliche Doppler-(Gefäß-) Information unmittelbar visuell zur Verfügung steht. Diese gestattet dann nicht nur das unmittelbare Erfassen der Position und Tiefe eines Blutgefäßes mittels der abgestuften, mehrkanaligen Doppler-Ausgabe (dieses wäre rein optisch durch das Endoskop nicht erkennbar, da die Blutgefäße innerhalb einer jeweiligen Organwand verborgen liegen), darüber hinaus ist sowohl der Blutfluß als auch das Vorliegen etwaiger Störkörper (z.B. Embolien) im Blutfluß mittels der farblich unterschiedenen Amplituden eines jeweiligen Frequenzabschnitts im Dopplerbild optisch unmittelbar erfaßbar. Die Referenz-Farbskala 30 gestattet diesbezüglich die quantitative Beurteilung eines derartigen, farblich hervorgehobenen Objektes. Mit dem kleinen Bildausschnitt kann jederzeit die Lage der Sonde kontrolliert und ggf. korrigiert werden, bis ein Signal gefunden wurde oder die Gewißheit besteht, daß kein Gefäß vorhanden ist.

Auf die vorbeschriebene Weise ist somit eine bislang unmögliche Untersuchung von Indikationen möglich, die etwa im Zusammenhang mit Blutungen aus dem Gastrointestinaltrakt in Zusammenhang stehen. Dies sind u.a. Ulcera, Varizen, Angiodysplasien oder auch Interventionen, wie z.B. die Polypektomie großer Polypen oder eine Tumor-Resektion. Das Ergebnis einer kombinierten Endoskopie-Doppleruntersuchung von derartigen, blutungsgefährdeten Stellen ermöglicht dann eine Entscheidung über weitere therapeutische Maßnahmen, etwa eine lokale Therapie, oder aber eine Feststellung, daß die Durchführung einer Operation nicht notwendig ist. Eine etwaige Intervention/Operation könnte dann in der ansonsten bekannten Weise unmittelbar über den Arbeits- bzw. Instrumentenkanal des Endoskops erfolgen.

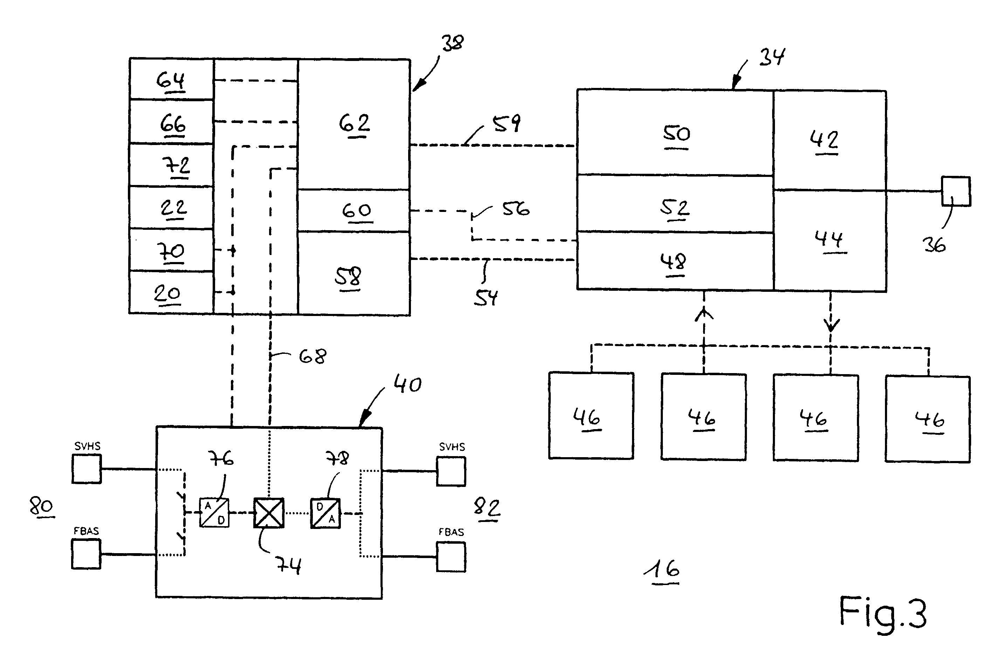

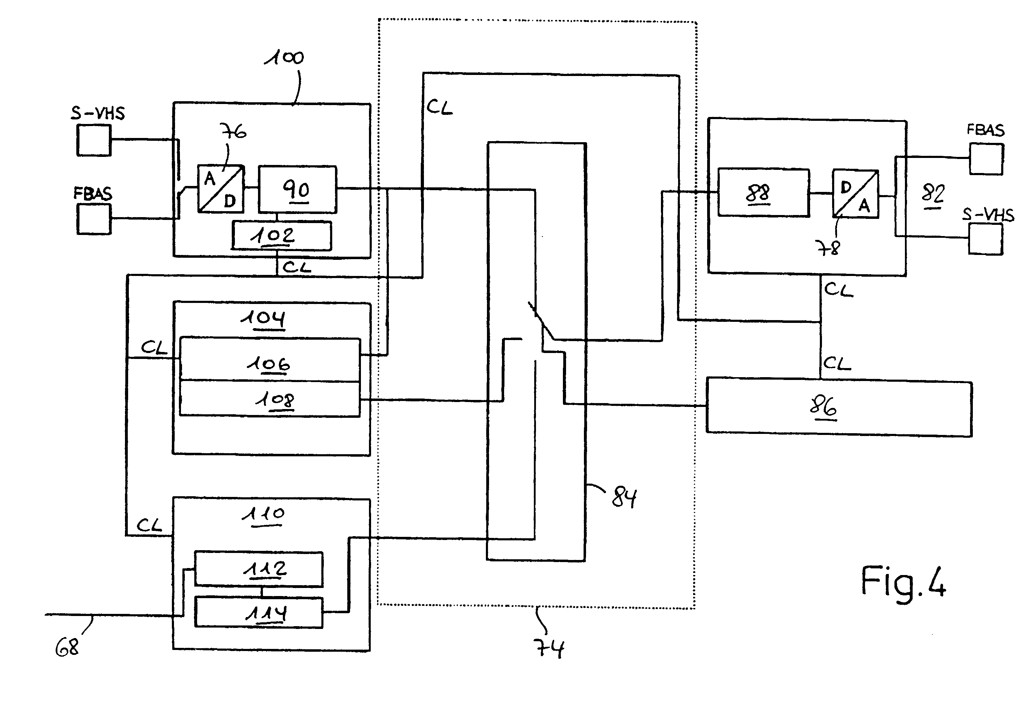

Unter Bezug auf die Fig. 3 und 4 wird im weiteren der Detailaufbau der schematischen, in Fig. 1 gezeigten Dopplerund Videoeinheit 16 beschrieben.

Diese besteht, wie in der Fig. 3 dargestellt, aus drei Funktionseinheiten -- einer Dopplereinheit 34, an welche über eine Sondenbuchse 36 die Ultraschallsonde 14 anschließbar ist, einer zentralen Steuereinheit 38, welche das aufbereitete Ausgangssignal der Dopplereinheit 34 empfängt und weiterverarbeitet, sowie einer Video-Verarbeitungseinheit 40, welche zum einen ein aufbereitetes Video-Dopplersignal der Steuereinheit 38 empfängt, und welche zum anderen Signaleingänge für das von der Endoskopie-Basiseinheit 12 erzeugte Endoskopie-Bildsignal aufweist.

Im einzelnen weist die Dopplereinheit 34 ein Sendemodul 42 sowie ein Empfangsmodul 44 auf, welche über die mit beiden Modulen verbundene Sonde 14 ein (z.B. 16 MHz) Doppier-Ultraschallsignal auf die interessierende Körperstelle bringen bzw. das von dort reflektierte Signal empfangen. Besonders vorteilhaft wird durch entsprechende Taktung bzw. Verzögerung auf der Empfangsseite (Gating) eine Mehrzahl von reflektierten Signalen eines -- bevorzugt gemeinsamen --Sendesignals empfangen, welches dann, bedingt durch die durch die Zeitverzögerung erreichten Laufzeitunterschiede, einer jeweiligen Eindringtiefe entspricht. Mit anderen Worten, bei dem konkret dargestellten, achtkanaligen Ausführungsbeispiel wird das sendeseitig ausgestrahlte Ultraschallsignal über das Empfangsmodul 44 zeitversetzt zu acht verschiedenen, aufeinanderfolgenden Empfangszeitpunkten aufgenommen, wobei die Zeitverzögerung zwischen Sendeimpuls und Empfang eines jeweiligen Empfangssignals der doppelten Signallaufzeit (Hin- und Rückweg) für die jeweilige Eindringtiefe entspricht. Auf diese Weise wird dann das achtkanalige Empfangssignal erhalten, welches mittels jeweils doppelt (zweikanalig) vorgesehener Mischereinheiten 46 in der ansonsten bekannten Weise demoduliert bzw. auf eine weiterverarbeitbare Endfrequenz umgesetzt wird. Das demodulierte, achtkanalige Empfangssignal wird dann einer Multiplexereinheit 48 zugeleitet, welche das achtkanalige Signal zur gemeinsamen, weiteren Übertragung und Verarbeitung zusammenfaßt und nachfolgenden Funktionsblöcken zur Verfügung stellt.

Gleichzeitig erfolgt über eine Prozessoreinheit 50 bzw. eine mit dieser zusammenwirkenden RAM-Steuereinheit 52 eine Prozessablaufsteuerung und -kontrolle des Dopplerbetriebes mit den vorbeschriebenen Einheiten.

Die Multiplexereinheit 48 stellt über eine analoge Audiosignalleitung 54 und eine analoge Bildsignalleitung 56 das demodulierte Dopplersignal zum einen einer Audio-Ausgabeeinheit 58 der Steuereinheit 38 zur Verfügung, und zum anderen einer A/D-Wandlereinheit 60 zur nachfolgenden digitalen Weiterverarbeitung des Bildsignals. Steuersignale fließen über eine separate Steuersignalleitung 59.

Während die Audio-Ausgabeeinheit 58 zur Verbindung mit einem (nicht gezeigten) Audio-Schallwandler, etwa einem Kopfhörer oder Lautsprecher, zur Ausgabe des demodulierten, in den NF-Bereich umgesetzten Dopplersignals (kanalweise einzeln oder über alle Kanäle summiert) vorgesehen ist (dies entspricht der traditionellen und nach wie vor gebräuchlichen Art der Verwendung einer Doppler-Ultraschalleinheit durch eine geschulte Bedienperson, die aus charakteristischen Tönen der Audioeinheit diagnostische Rückschlüsse vornehmen kann), ist der A/D-Wandler 60 einem Signalprozessor 62 vorgeschaltet, welcher das digitale Bildsignal einer rechnerischen Verarbeitung unterzieht. Insbesondere wird an dieser Stelle durch Programmsteuerung aus einem in einem Flash-EPROM 64 abgelegten Programm bzw. in Zusammenwirken mit einem Arbeitsspeicher 66 (bevorzugt als SRAM realisiert) eine kontinuierliche und periodische (Fast-) Fourier-Transformation der jeweiligen, kanalweisen Bildsignale dergestalt vorgenommen, daß die in der Fig. 2 mit dem Bezugszeichen 28 angedeuteten Signaldarstellungen der jeweiligen Frequenz als Funktion der Zeit bei farblich herausgehobener Amplitude entstehen. Mit anderen Worten, mittels der Fourier-Transformation wird das Ultraschall-Dopplersignal vom Zeit- in den Frequenzbereich umgesetzt, die einem jeweiligen Zeitpunkt empfangenen (Frequenz-) Spektrallinien werden berechnet und entlang des Verlaufs einer jeweiligen Spektrallinie abschnittsweise im Hinblick auf ihre Signalamplitude für die farbliche Unterscheidung gemäß Referenzskala 30 quantifiziert.

Das so entstehende, mehrkanalige und in die gewünschte Darstellungsform transformierte Dopplersignal wird dann über eine Doppler-Bilddatenleitung 68 der Video-Verarbeitungseinheit 40 bereitgestellt. Darüber hinaus sind digitale (Steuer-) Signalleitungen vorgesehen, die zum einen den Signalprozessor 62 mit der Steuereinheit 50 der Dopplereinheit 34 verbinden, und zum anderen den Signalprozessor 62 mit der Video-Verarbeitungseinheit 40. Über diese Leitungen werden die Prozessschritte der jeweils beteiligten Funktionsmodule gesteuert und koordiniert.

Zusätzlich weist die Steuereinheit 38 noch den in der Fig. 1 gezeigten Fußschalter 20 als schematischen Funktionsblock dargestellt auf. Ebenfalls sind weitere, ggf. notwendige Bedienelemente 70 vorgesehen, sowie eine serielle Schnittstelle bzw. Fernbedienungseinheit 22 und ein geeigneter Drucker 72, welcher etwa in ansonsten bekannter Weise durch ein integriertes Thermodruckmodul realisiert sein kann.

Prinzipiell besteht die in der Fig. 3 gezeigte Video-Verarbeitungseinheit 40 aus einem zentralen Video-Mischer 74, der digital arbeitet und beidseitig mit entsprechenden A/D- bzw. D/A-Wandlern 76, 78 verbunden ist. Eingangsseitig empfängt der A/D-Wandler 76 das Endoskop-Bildsignal der Endoskopie-Basiseinheit 12 über einen (umschaltbaren) Video-Eingangsport 80, und ausgangsseitig gibt der D/A-Wandler 78 das Video-Mischsignal über entsprechend normierte Video-Ausgänge 82 an einen anzuschließenden Monitor aus.

Zur Verdeutlichung der Funktionsweise der Video-Verarbeitungseinheit 40 wird nachfolgend auf die weiter detaillierte Darstellung der Fig. 4 eingegangen.

Hier wird erkennbar, daß die zentrale Video-Mischeinheit 74 als Multiplexer 84 realisiert ist, der als Reaktion auf eine zugeordnete Multiplexer-Steuereinheit 86 zwischen drei Video-Signalquellen umschaltet bzw. wechselt und das resultierende Signal dem ausgangsseitigen D/A-Wandler 78, welchem ein Video-Codierer (Encoder) 88 zur Normbildgenerierung (z. B. PAL, NTSC, SECAM) vorgeschaltet ist, bereitstellt. Auch kann die Möglichkeit zur Verarbeitung von RGB-Signalen vorgesehen sein.

Alternativ oder zusätzlich ist es möglich, Bilddaten in Form von Grafik-Bildsignalen zu verarbeiten, wie sie etwa gängigen (PC-) Grafikstandards entsprechen. Besonders bevorzugt sind also die bildverarbeitenden Komponenten der Erfindung in entsprechender Weise grafikfähig.

Für die Behandlung des Endoskop-Video-Eingangssignals ist diesbezüglich dem eingangsseitigen A/D-Wandler 76 ein entsprechender Videonorm-Decoder 90 nachgeschaltet, dessen Ausgangssignal als erster Kanal am Multiplexer 84 anliegt.

Bestandteil der Endoskop-Video-Eingangseinheit 100 ist zudem ein Systemtaktgenerator 102, der an alle Funktionsbaugruppen der Video-Verarbeitungseinheit in Fig. 4 einen gemeinsamen Systemtakt CL anlegt.

Das Ausgangssignal des Decoders 90 wird auch von einer Video-Skaliereinheit 104 empfangen, welche das eingehende (Vollbild-) Endoskop-Bild in das beabsichtigte Endformat -beispielsweise die Fensterdarstellung 26 in Fig. 2 -- umsetzt. Zu diesem Zweck weist die Skaliereinheit 104 eine Eingangsformatiereinheit 106 sowie eine Ausgangsformatiereinheit 108 auf. Eine Video-Controllereinheit 110 für das von der Steuereinheit 38 eingehende, digitale Dopplerbild-Signal (zur Vereinfachung ist die Doppler-Bilddatenleitung 68 mit der parallelen Steuerdatenleitung zusammengefaßt dargestellt) bildet den nach der Video-Skaliereinheit 104 (zweiter Kanal) dritten Eingangskanal für den Multiplexer 84. Die Video-Controllereinheit 110 weist, wie dargestellt, eine Steuereinheit 112 auf, welche mit einem Video-RAM 114 zusammenwirkt.

Der Multiplexer 84 schaltet nun -- je nach vorgewähltem Darstellungsmodus -- zwischen den Bild-Eingangsquellen um bzw. wechselt zwischen diesen, so daß ausgangsseitig die beabsichtigten Darstellungen entstehen: Entweder das Endoskop-Bild in einer Ganzbilddarstellung oder aber eine fensterweise unterteilte Einzelbilddarstellung der einzelnen Ultraschall-Dopplerkanäle, in vorbeschrieben dargestellter Weise gemischt mit einem herunterskalierten Endoskopie-Bild. Während für den ersten Fall das Endoskop-Eingangsbildsignal über den Multiplexer 84 unmittelbar auf den Ausgang durchgeschleift wird, wird bei gemischter Darstellung, d.h. verkleinertes Endoskopie-Bild plus Doppler-Darstellung, mit hoher Taktzahl zwischen den entsprechenden Eingangsquellen umgeschaltet, so daß ausgangsseitig dann das Mischsignal entsteht.

Die beschriebene Anordnung erlaubt dann das einfache Zusammenführen der verschiedenen, untersuchungsrelevanten Bildinformationen zur unmittelbaren Nutzung durch eine Bedienperson auf einen Blick. Somit kann dann nicht nur die Bedienperson -- etwa ein Endoskopeur/Operateur -- von ablenkenden Bedienungsschritten entlastet werden; darüber hinaus ermöglicht die neuartige, zusammengeführte Bilddarstellung erstmals auch eine Vorgehensweise beim Betrieb, die komplexeren Diagnose- und Behandlungsaufgaben gerecht wird.

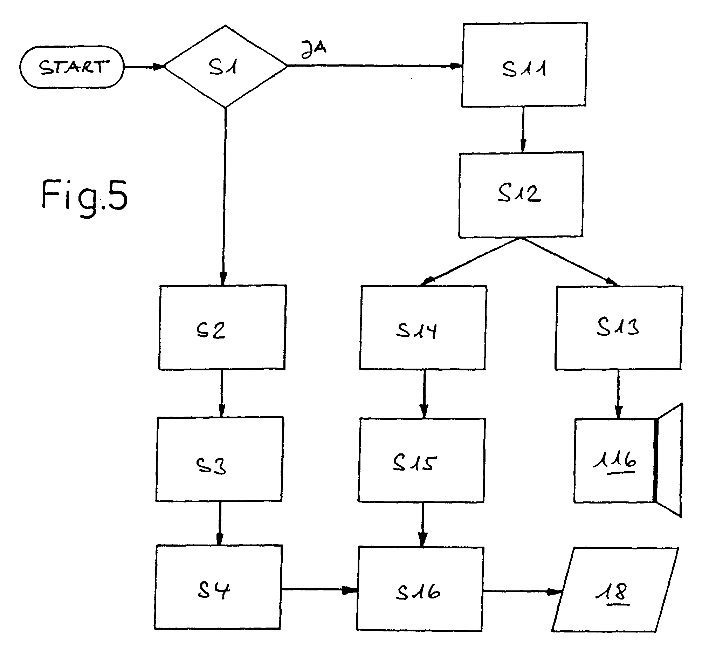

Ein Betriebsverfahren der vorstehend beschriebenen Vorrichtung wird im weiteren unter Bezug auf die Fig. 5 beschrieben.

Nach dem Verfahrensstart wird entschieden, ob in die beabsichtigte Video-Bilddarstellung das (mehrkanalige) Doppler-Signal einzublenden ist (S1). Wenn dies zu bejahen ist -etwa, weil der Fußschalter 20 in einer entsprechenden Schaltposition steht -- wird in Schritt S11 das hochfrequente Doppler-Sendesignal erzeugt (Sendemodul 42) und über die angeschlossene Ultraschallsonde 14 abgestrahlt. Das reflektierte Signal wird, entsprechend einer jeweiligen Eindringtiefe, mehrkanalig empfangen und gemischt (Schritt S12).

Einerseits findet dann eine Audiosignalverstärkung und-ausgabe auf eine Lautsprechereinheit 116 statt (Schritt S13), andererseits wird das analoge Bildsignal dann mehrkanalig in ein Digitalsignal umgesetzt (Schritt S14), in Schritt S15 digital weiterbearbeitet und aufbereitet(Fourier-Transformation) und in Schritt 16 mit dem digitalisierten Endoskop-Signal in der vorbeschriebenen Weise gemischt.

Dieses Endoskopie-Signal wurde in Schritt S2 mittels des Videoendoskops erzeugt, in Schritt S3 kodiert und dann digitalisiert (S4). Das in Schritt S16 gemischte Bildsignal wird schließlich an die Video-Ausgabeeinheit 18 ausgegeben.

Für den Fall, daß lediglich eine Vollbild-Darstellung des Endoskop-Bildes gewünscht ist (Entscheidung in Schritt S1: Nein), findet lediglich ein Betrieb entlang der Abfolge S2-S3-S4-S16 statt, wobei während des Mischens in Schritt S16 dann eine entsprechende Vollbild-Signalquelle auf die Video-Ausgabeeinheit geschaltet wird (vgl. die Darstellung in Fig. 4). Auch ist es möglich, grundsätzlich das Endoskopiebildsignal erst beim Einschalten des Dopplergeräts auf den Mischer zu legen und ansonsten diesen zu umgehen; hierdurch könnte durch Trennung der Bildsignale die Bildqualität verbessert werden.

Erfindungsgemäß ist es somit möglich, das sich aus dem Stand der Technik stellende Problem der gleichzeitigen Überwachung eines Endoskop-Bildes bei zusätzlich notwendiger Überprüfung auf eventuell vorhandene Blutgefäße zu lösen, so daß die Bedienperson in entsprechend größerer Weise ihre Aufmerksamkeit auf die endoskopische Untersuchung richten kann. Gleichzeitig wird erfindungsgemäß eine genaue und zuverlässige Information über das Vorhandensein und eine etwaige Tiefe eines (Blut-) Gefäßes gegeben, wobei hierfür insbesondere die Ausführungsform mit den beschriebenen acht Kanälen eine gute Informationsbasis bietet.

Dabei ist sowohl eine jeweilige Untraschalleistung als auch weitere Parameter, etwa die Abstufung der Eindringtiefen, je nach gewünschtem Einsatzzweck vorwähl- bzw. einstellbar. Auch ist weiterbildungsgemäß vorgesehen, etwa mittels eines Auswahlmenüs der wichtigsten Indikationen eine jeweilige, vorgegebene Parameterkonfiguration aufzurufen und einzustellen.

Soll beispielsweise bei einer Ulcus-Untersuchung nach einem potentiellen Geschwür in Magen oder Darm endoskopisch gesucht werden, wird geeignet zur Erfassung möglicher Blutgefäße eine in 0,3 mm abgestufte Eindringtiefe der acht Empfangskanäle zwischen etwa 0,3 und etwa 2,4 mm eingestellt. Die horizontale Zeitachse der Darstellung des Ultraschall-Doppler-Signals ist mit 6 Sekunden für 4 bis 6 Herzzyklen ausreichend; selbstverständlich liegt es auch hier im Belieben der Bedienperson (bzw. des einschlägigen Fachmannes), situationsbedingt eine Einstellung vorzunehmen.

Die vorliegende Erfindung ist weder auf eine Verwendung mit acht Ultraschall-Dopplerkanälen beschränkt -- vielmehr ist es auch hier möglich, je nach beabsichtigtem Einsatzzweck eine benötigte Kanalzahl, beispielsweise etwa zwischen 1 und 16, einzusetzen, und auch die beschriebene Bilddarstellung bzw. -anordnung in Fig. 2 ist als rein exemplarisch zu verstehen. Zwar hat es sich in der praktischen Bedienung bewährt, für eine Bedienperson die Möglichkeit einer manuellen Beeinflussung der Anordnung auf dem Bildschirm weitgehend einzuschränken, um hier kein zusätzliches Ablenkungspotential zu schaffen; gleichwohl liegt es im Ermessen des Durchschnittsfachmannes, etwaige Bild- und Fensteranordnungen auf dem Bildschirm nach Belieben zu gestalten oder technisch zu realisieren. Hierzu würde auch das selektive (Heraus-) Vergrößern einzelner Dopplerdarstellungen zählen. Auch könnte grundsätzlich eine akustische Ausgabe des Dopplersignals permanent erfolgen.

Auch richtet sich die Auswahl einer geeigneten Dopplersonde nach den durch ein vorhandenes Endoskop vorliegenden Möglichkeiten. So hat es sich in der Praxis herausgestellt, daß derartige endoskopische Dopplersonden mit ihren Zuleitungen einen Außendurchmesser von 2,6 mm (für die flexible Endoskopie) zur Einführung in den entsprechenden Instrumentierkanal nicht überschreiten sollten. Auch hier ist jedoch eine Ausgestaltung je nach Bedarf möglich.

Weiterbildungsgemäß liegt es im Rahmen der Erfindung, eine genaue Plazierung bzw. Justierung der Doppler-Sondenposition vor Ort vorzunehmen: Diese ragt einen gewissen Abstand aus dem Instrumentierkanal heraus und kann durch geeignete Manipulationen, etwa einer zusätzlich induzierten Bewegung des Sondenkopfes, gegenüber den umgebenden Organwänden positioniert werden. Gerade im Hinblick darauf, daß eine dopplersonographische Erfassung bei einer Sondenposition i.w. tangential zu einem zu beobachtenden Gefäß besonders effektiv ist, liegen in dieser weiterbildungsgemäßen Technologie noch beträchtliche Verbesserungs- und Optimierungspotentiale.

Neben einer solchen Sondenbewegung, die etwa in der Art eines bekannten, steuerbaren Katheters mechanisch erfolgen könnte, wäre es möglich, eine rotierende Sonde einzusetzen, oder aber eine elektrische oder linsentechnische Beeinflussung des Sonden-Erfassungsbereiches (bzw. dessen Schallfeldes) durch geeignete schaltungstechnische Maßnahmen vorzunehmen.

Die vorliegende Erfindung ist nicht auf die vorbeschriebene Ausführungsform einer Verwendung eines Endoskops im Zusammenhang mit der Ultraschallerfassung von Körpergefäßen beschränkt.

So ist es insbesondere auch eine geeignete (in den Fig. nicht gezeigte) Ausführungsform, die Erfindung im Zusammenhang mit Mikroskopen od.dgl. Instrumenten zu verwenden, deren Bild von einem Bilderfassungssensor -- etwa einem CCD-Element -- erfaßt und zur Weiterverarbeitung im Wege der erfindungsgemäßen Bildmischung verwendet wird. Damit gäbe es etwa die Möglichkeit einer Anwendung eines solchen Videomikroskops in der Neurochirurgie dergestalt, daß ein Mikroskop vor das Operationsfeld plaziert wird, durch das der Operateur hindurchschaut. Das Mikroskopbild, erfaßt von dem CCD-Sensor, wird dann über eine geeignete Video-Bildaufbereitung auf einem Monitor dargestellt, wahlweise zu Mischen mit einem Ultraschall-Dopplersignal, welches durch eine Ultraschallsonde erzeugt wird. Diese Ultraschallsonde kann entweder an dem Bilderfassungselement bzw. dem zugehörigen Träger (hier: Mikroskop) sitzen.

## Patentansprüche

1. Vorrichtung für die Beobachtung von Gefäßen, insbesondere von Blutgefäßen, in Körperöffnungen, - höhlen und Hohlorganen mit
einem an einer in eine Beobachtungsposition führbaren Trägereinrichtung (10) gehaltenen Bilderfassungselement und einem bevorzugt eingriffseitig an der Trägereinrichtung (10) vorgesehenen Ultraschallwandler (14),
einer mit dem Ultraschallwandler (14) verbindbaren, auf dem Dopplerprinzip beruhenden Ultraschalleinheit (34), die zum Erfassen einer Organ- und/oder Fluidbewegung in mindestens einem vorbestimmten Abstand von dem Ultraschallwandler ausgebildet ist, **gekennzeichnet durch**
eine der Ultraschalleinheit (34) nachgeschaltete Doppler-Auswerteinheit (38), die zum digitalen Umsetzen eines Ausgangssignals der Ultraschalleinheit (34) in eine visuelle Darstellung der Organ- und/oder Fluidbewegung in dem mindestens einen vorbestimmten Abstand ausgebildet ist,
eine ein Ausgangs-Bildsignal der Doppier-Auswerteinheit (38) und ein elektronisches Bildsignal des Bilderfassungselements empfangende, eine Video-Mischereinheit (7) aufweisende Video-Verarbeitungseinheit (40), die zum Erzeugen eines kombinierten Gesamtbildes (24) für einen Bildschirm ausgebildet ist, das sowohl ein erfaßtes Bild (26) als auch mindestens eine Darstellung (28) der Organund/oder Fluidbewegung zeigen kann und eine auf einen Betrieb der Video-Verarbeitungseinheit (40) wirkende Moduswahleinrichtung (20) in Form eines Umschalters, der zum Umschalten von einem ersten Betriebsmodus mit einem erfassten Vollbild des Bilderfassungselements, insbesondere einem Endoskopie-Vollbild, in einen zweiten Betriebsmodus mit dem kombinierten Gesamtbild auf dem Bildschirm ausgebildet ist.

2. Vorrichtung nach Anspruch 1, **dadurch gekennzeichnet, daß** die Trägereinrichtung (10) mit dem Bilderfassungselement durch ein Endoskop realisiert ist.

3. Vorrichtung nach Anspruch 1, **dadurch gekennzeichnet, daß** die Trägereinrichtung als Operationsmikroskop realisiert ist.

4. Vorrichtung nach Anspruch 1 oder 2, **dadurch gekennzeichnet, daß** der Ultraschallwandler (14) in einem Arbeits- oder Instrumentenkanal eines Endoskops (10) in eine endseitige Betriebsposition führbar ausgebildet ist.

5. Vorrichtung nach einem der Ansprüche 1 bis 4, **dadurch gekennzeichnet, daß** die Ultraschalleinheit (34) mittels mehrkanalig vorgesehener Funktionsblöcke zum Erfassen der Organ- und/oder Fluidbewegung in einer Mehrzahl von verschiedenen Eindringtiefen entsprechenden Abständen ausgebildet ist.

6. Vorrichtung nach Anspruch 5, **dadurch gekennzeichnet, daß** die Ultraschalleinheit (34) eine steuerbare Verzögerungseinrichtung aufweist, die die Mehrzahl von Eindringtiefen auf der Basis einer jeweiligen Signallaufzeit bestimmt.

7. Vorrichtung nach einem der Ansprüche 1 bis 6, **dadurch gekennzeichnet, daß** auf dem Gesamtbild, entsprechend einer erfaßten Mehrzahl von vorbestimmten Abständen zum Ultraschallwandler, eine Mehrzahl von Bewegungsdarstellungen (28ᵢ) gezeigt werden kann.

8. Vorrichtung nach Anspruch 7, **dadurch gekennzeichnet, daß** die Mehrzahl von Bewegungsdarstellungen fensterartig und in einer Abfolge einer jeweiligen Eindringtiefe angeordnet werden kann.

9. Vorrichtung nach einem der Ansprüche 1 bis 8, **dadurch gekennzeichnet, daß** die Moduswahleinrichtung als Fernbedienungseinheit, insbesondere als Fußschalter (20) realisiert ist.

10. Vorrichtung nach einem der Ansprüche 1 bis 9, **dadurch gekennzeichnet, daß** die Video-Verarbeitungseinheit (40) eine digitale Videomischeinrichtung (74) aufweist.

11. Vorrichtung nach einem der Ansprüche 1 bis 10 **dadurch gekennzeichnet, daß** die Video-Verarbeitungseinheit eine digitale Skaliereinheit (104) für das erfaßte Bild des Bilderfassungselements aufweist.

12. Vorrichtung nach einem der Ansprüche 1 bis 11, **gekennzeichnet durch** einen Decoder und/oder Endcoder (88,90) für TV-Bildsignale, insbesondere PAL, NTSC, SECAM.

13. Vorrichtung nach einem der Ansprüche 1 bis 12, **dadurch gekennzeichnet, daß** der Ultraschallwandler (14) Bestandteil einer Sondeneinrichtung ist, die eine mechanische Positioniereinrichtung zur Veränderung einer Lage des Ultraschallwandlers (14) an der Beoachtungsposition aufweist.

14. Vorrichtung nach einem der Ansprüche 1 bis 13, **dadurch gekennzeichnet, daß** der Ultraschallwandler (14) mit einer Einrichtung zur gesteuerten, insbesondere elektronischen Veränderung des Wandler-Schallfeldes versehen ist.

## Claims

1. Device for observing vessels, in particular blood vessels, in orifices of the body, cavities of the body and hollow organs, comprising an image detecting element held on a carrying device (10) guidable into an observation position and an ultrasonic transducer (14) provided preferably at the engagement side of the carrying device (10), an ultrasonic unit (34) based on the Doppler principle and connectable to the ultrasonic transducer (14), the ultrasonic unit being constructed for detecting an organ and/or a fluid movement ac at least one predetermined distance from the ultrasonic transducer, **characterised by** a Doppler evaluation unit (38) connected downstream of the ultrasonic unit (34) and constructed for digitally converting an output signal of the ultrasonic unit (34) into a visual display of the organ and/or fluid movement at the at least one predetermined distance, a video processing unit (40) receiving an output image signal of the Doppler evaluation unit (38) and an electronic image signal of the image detecting element and comprising a video mixing unit (7), the processing unit (40) being constructed to produce a combined overall image (24) for a screen which can display both a detected image (26) and at least one depiction (28) of the organ and/or fluid movement, and a mode selecting device (20) in the form of a changeover switch acting on operation of the video processing unit (40), the changeover switch being constructed for changing over from a first operating mode with a detected picture of the image detecting element, in particular an endoscopy picture, into a second operating mode wich the combined overall image on the screen.

2. Device according to claim 1, **characterised in that** the carrying device (10) with the picture detecting element is produced by an endoscope.

3. Device according to claim 1, **characterised in that** the carrying device is produced as an operating microscope.

4. Device according to claim 1 or 2, **characterised in that** the ultrasonic transducer (14) in an operating or instrument duct of an endoscope (10) is constructed so as to be guidable into an end operating position.

5. Device according to any of claims 1 to 4, **characterised in** chat the ultrasonic unit (34) is designed by means of functional blocks provided with multiple ducts for detecting the organ and/or fluid movement at a plurality of distances corresponding to different penetration depths.

6. Device according to claim 5, **characterised in that** the ultrasonic unit (34) comprises a controllable delay unit which determines the plurality of penetration depths on the basis of a respective signal running time.

7. Device according to any of claims 1 to 6, **characterised in that** a plurality of movement depictions (28₁) can be displayed on the overall image, in accordance with a detected plurality of predetermined distances from the ultrasonic transducer.

8. Device according co claim 7, **characterised in that** the plurality of movement depictions can be arranged in a window-like manner and in a sequence of a respective penetration depth.

9. Device according to any of claims 1 to 8, **characterised in** char the mode selecting device is produced as a remote concrol unit, in particular as a foot switch (20).

10. Device according to any of claims 1 to 9, **characterised in that** the video processing unit (40) comprises a digital video mixing device (74)

11. Device according to any of claims 1 to 10, **characterised in that** the video processing unit comprises a digital scaling unit (104) for the detected image of the image detecting element.

12. Device according to any of claims 1 to 11, **characterised by** a decoder and/or encoder (88, 90) for TV image signals, in particular PAL, NTSC, SECAM.

13. Device according to any of claims 1 to 12, **characterised in that** the ultrasonic transducer (14) is a component of a probe device comprising a mechanical positioning device for altering a position of the ultrasonic transducer (14) at the observation position.

14. Device according to any of claims 1 to 13, **characterised in that** the ultrasonic transducer (14) is provided with a device for controlled, in particular electronic, alteration of the transducer sound field.

## Revendications

1. Dispositif pour l'observation de vaisseaux, particulièrement de vaisseaux sanguins dans les orifices et cavités du corps et les organes creux avec :
- Un élément d'enregistrement d'image maintenu dans une position d'observation par un dispositif de support maniable (10) et un transducteur à ultrasons (14), de préférence prévu sur la face opérationnelle du dispositif de support (10) ;
- Une unité échographique (34) basée sur l'effet Doppler, reliable au transducteur à ultrasons (14), conçue pour l'enregistrement du mouvement d'organe et/ou de fluide à au moins une distance prédéterminée du transducteur à ultrasons, **caractérisée en ce que** :
- Une unité d'examen Doppler (38) en aval de l'unité échographique (34), est conçue pour la conversion d'un signal de sortie de l'unité échographique (34) en une représentation visuelle du mouvement d'organe et/ou de fluide à au moins la distance prédéterminée ;
- Une unité de traitement vidéo (40) présentant une unité de montage vidéo (7), recevant un signal de sortie d'image de l'unité d'examen Doppler (38) et un signal d'image électronique de l'élément d'enregistrement d'image, est conçue pour générer une image combinée (24) pour un moniteur, qui peut aussi bien montrer une image enregistrée (26) qu'au moins une représentation (28) du mouvement d'organe et/ou de fluide, et un dispositif de sélection de mode (20) en forme de commutateur agissant sur un mode de l'unité de traitement vidéo (40), conçu pour la commutation d'un premier mode de fonctionnement avec une image enregistrée de l'élément d'enregistrement d'image, plus particulièrement une image d'endoscopie, en un second mode de fonctionnement avec l'image combinée sur le moniteur.

2. Dispositif selon la revendication 1, **caractérisé en ce que** le dispositif de support (10) avec l'élément d'enregistrement d'image est réalisé sous la forme d'un endoscope.

3. Dispositif selon la revendication 1, **caractérisé en ce que** le dispositif de support est réalisé comme un microscope opératoire.

4. Dispositif selon la revendication 1 ou 2, **caractérisé en ce que** le transducteur à ultrasons (14) est conçu pour être manipulé dans une position de travail terminale dans un canal opérateur d'un endoscope (10).

5. Dispositif selon une des revendications 1 à 4, **caractérisé en ce que** l'unité échographique (34) est formée au moyen de blocs fonctionnels multicanaux pour enregistrer le mouvement d'un organe et/ou d'un fluide à un nombre de distances correspondant aux différentes profondeurs de pénétration.

6. Dispositif selon la revendication 5, **caractérisé en ce que** l'unité échographique (34) présente un dispositif de temporisation pilotable qui définit le nombre des profondeurs de pénétration sur la base d'un temps de propagation du signal respectif.

7. Dispositif selon une des revendications 1 à 6, **caractérisé en ce que** sur l'image globale, un nombre de représentations de mouvements (28ᵢ) peut être affiché, correspondant à un nombre saisi de distances prédéterminées au transducteur à ultrasons.

8. Dispositif selon la revendication 7, **caractérisé en ce que** les différentes représentations de mouvement peuvent être disposées en fenêtrage et dans l'ordre d'une profondeur de pénétration respective.

9. Dispositif selon une des revendications 1 à 8, **caractérisé en ce que** le dispositif de sélection de mode est réalisé comme une unité de commande à distance, plus particulièrement comme un interrupteur à pédale (20).

10. Dispositif selon une des revendications 1 à 9, **caractérisé en ce que** l'unité de traitement vidéo (40) présente un dispositif de montage vidéo numérique (74).

11. Dispositif selon une des revendications 1 à 10, **caractérisé en ce que** l'unité de traitement vidéo présente un affichage numérique gradué (104) pour l'image enregistrée de l'élément d'enregistrement d'image.

12. Dispositif selon une des revendications 1 à 11, **caractérisé par** un décodeur et/ou un encodeur (88, 90) pour les signaux de télévision, plus particulièrement PAL, NTSC, SECAM.

13. Dispositif selon une des revendications 1 à 12, **caractérisé en ce que** le transducteur à ultrasons (14) fait partie d'un dispositif de sonde, qui présente un dispositif de positionnement pour la modification d'une orientation du transducteur à ultrasons (14) à la position d'observation.

14. Dispositif selon une des revendications 1 à 13, **caractérisé en ce que** le transducteur à ultrasons (14) est équipé d'un dispositif pour la modification assistée, plus particulièrement par électronique, du champ sonore du transducteur.
